# EUROPEAN PATENT SPECIFICATION

(11) **EP 0 351 076 B1**
(45) Date of publication and mention of the grant of the patent: **18.08.1993**
(21) Application number: 89306295.0
(22) Date of filing: 22.06.1989
(51) Int. Cl.: A61K 6/00

(54) **Dental composition**
Dentalzusammensetzung
Composition dentaire

(30) Priority: 13.07.1988 JP 175627/88
(43) Date of publication of application: 17.01.1990
(73) Proprietor: KURARAY CO., LTD., Kurashiki-City (JP)
(72) Inventor: Omura, Ikuo, Kurashiki-City (JP)
(74) Representative: Thomas, Roger Tamlyn

(56) References cited:
- US-A- 4 340 529
- WPIL FILE SUPPLIER, AN=88-087146 [13], Derwent Publications Ltd, London (GB)#
- WPIL FILE SUPPLIER, AN 84-007214/02#

## Description

### BACKGROUND OF THE INVENTION

### Field of the invention

This invention relates to dental compositions, and more particularly, to dental compositions useful as a primer or an adhesive to be applied to the surface of teeth and/or dental materials to thereby effect adhesion to them.

### Description of the prior art

Generally used in the dental field is a method which comprises using a solution of a liquid methacrylate monomer and an organic peroxide such as dibenzoyl peroxide in a volatile organic solvent. The solution however tends to undergo polymerization in storage, and hence the storage of the solution requires special considerations. Thus, such solutions are usually kept in a container having a high oxygen permeability, such as polyethylene and polypropylene, and in a volume of as small as about 10 cm³, thereby facilitating a constant supply to the solvents, of oxygen gas which has an important function in preventing the solution from polymerizing. Typical volatile organic solvents such as hydrocarbons, esters and ketones having a relatively low molecular weight permeate readily through plastics having a high oxygen permeability. Therefore, when a solution of a methacrylate monomer diluted with such an organic solvent is kept in a plastics container, the organic solvent permeates through the container and evaporate resulting in a volume reduction of the solution.

Alcohols such as ethyl alcohol and isopropyl alcohol are known diluting solvents for dental priming agents or dental adhesives. Though such alcohols do not permeate through the above plastics, they have a drawback of remarkably accelerating the decomposition of the organic peroxides. A composition comprising a radical-polymerizable monomer and an organic peroxide, diluted with such an alcohol, therefore rapidly reduces its content of the organic peroxide or solidifies due to polymerization, and hence it has been impossible to store the solution stably for a long period of time. Accordingly, it has been hard to provide users with a composition comprising a radical-polymerizable monomer and an organic peroxide, which are dissolved in a volatile solvent, as a primer or adhesive for dental use.

### SUMMARY OF THE INVENTION

Accordingly, an aim of the present invention is to provide a dental composition, comprising a radical-polymerizable monomer, an organic peroxide and a volatile organic solvent (diluting agent).

The present inventor has, for achieving the above-described aim, made intensive studies to find a volatile organic solvent which hardly permeates through plastics having a high oxygen permeability and in which the organic peroxide decomposes only a little. As a result, tertiary alcohols, among a number of organic solvents, were found to satisfy, exceptionally, the above conditions, and the invention was completed.

Thus, the present invention provides dental compositions comprising a radical-polymerizable monomer and an organic peroxide dissolved in a tertiary alcohol, such dental compositions being packaged in an oxygen-permeable plastics container.

### DESCRIPTION OF THE PREFERRED EMBODIMENTS

In the present invention, any known radical-polymerizable monomer can be used but, preferably used are (meth)acrylate monomers generally used for dental materials [the term "(meth)acrylate" herein means either methacrylate or acrylate].

Examples of such monomer include methyl (meth)acrylate, ethyl (meth)acrylate, 2-hydroxyethyl (meth)acrylate, ethylene glycol di(meth)acrylate, neopentyl glycol di(meth)acrylate, triethylene glycol di(meth)acrylate, 2,2-bis[4-(3-methacryloyloxy-2-hydroxypropoxy)phenyl]propane (abbreviated as Bis-GMA), 2,2-bis[(meth)acryloyloxypolyethoxyphenyl]propane, trimethylolpropane tri(meth)acrylate, (meth)acrylic acid, 2-methacryloyloxyethyl dihydrogen phosphate, 10-methacryloyloxydecyl dihydrogen phosphate, bis[2-methacryloyloxyethyl] hydrogen phosphate, 4-[2-methacryloyloxyethoxycarbonyl]phthalic acid anhydride (popular name: 4-META), 4-[2-methacryloyloxyethoxycarbonyl]phthalic acid (popular name: 4-MET), 11,11-dicarboxyundecyl(meth)acrylate, 3-methacryloyloxypropyltrimethoxysilane, and 2-(N,N-dimethylamino)ethylmethacrylate.

Examples of the organic peroxide used in the present invention include such known organic peroxides as dibenzoyl peroxide, di-p-chlorobenzoyl peroxide, di-2,4-dichlorobenzoyl peroxide, tertiary butyl peroxybenzoate, methylethyl ketone peroxide, ditertiary butyl peroxide, dicumyl peroxide and cumene hydroperoxide.

The gist of the present invention lies in the use of volatile tertiary alcohols. The term "volatile" as used herein means "having a boiling point of not more than 200°C under atmospheric pressure". Examples of such alcohols are tert-butanol (b.p. 82.5°C), tert-pentanol (b.p. 101.8°C), 3-methyl-3-pentanol (b.p. 126°C), 3-ethyl-3-pentanol (b.p. 143°C), 1-methylcyclohexanol (b.p. 155°C), 2-methyl-2-heptanol (b.p. 156°C), 3-methyl-3-heptanol (b.p. 159°C), and 4-methyl-4-heptanol (b.p. 161°C). One or more of the hydrogen atoms bonded to the carbon atoms of these alcohols may be substituted with a halogen(s) such as Br or Cl or acyl group(s). These tertiary alcohols are used either singly or in admixtures of at least two.

The above tertiary alcohols may also be used as mixtures with another organic solvent not having radical-polymerizability. In this case, it is preferred that the other organic solvents be mixed in a ratio less than 1 part be weight based on one part by weight of the tertiary alcohols, and that such other organic solvent be one which does not accelerate the decomposition of the organic peroxide used.

When using the composition of the present invention as a dental primer, the preferred composition ratio is 0.1 to 300 weight parts of the radical-polymerizable monomer and 0.01 to 10 weight parts of the organic peroxide based on 100 weight parts of the diluting solvent.

The primer generally incorporates such ingredients as a polymerization inhibitor and an antioxidant, to thereby prevent polymerization in storage. Sometimes a photopolymerization initiator such a camphorquinone or benzil may also be incorporated.

The dental compositions of the present invention are usually supplied in a plastics container. Oxygen gas should permeate through the plastic to thereby prevent the monomer from polymerizing, and the oxygen permeability coefficient is preferably at least 0.3 x 10⁻¹⁰ cm²/sec·kPa (0.4 x 10⁻¹⁰ cm²/sec. (cmHg)). Examples of such plastics include polyethylene, polypropylene and poly (4-methylpentene-1).

According to the present invention, dental compositions which are improved in the reduction of solvent evaporation and decomposition of the organic peroxide can be obtained by dissolving a radical-polymerizable monomer and an organic peroxide in a volatile tertiary alcohol.

The dental compositions of the present invention can be supplied to the users (dentists and dental technicians) while packed in a drop-type container having the shape of an eye-lotion container. The users can take out the required amount of the composition by simply pinching the container. Then, the users can apply the composition to the surface of a tooth and/or a dental material to be treated, allow the diluting solvent to evaporate and form a thin coated layer of the composition on the surface.

The dental composition of the present invention is applied as a single-package primer to the surface of the dental metal or the tooth, whereby the metal or the tooth is firmly bonded to other dental materials. The composition can also be used as a counterpart of a two-package adhesive, wherein it is mixed with another solution containing a reducing agent (e.g. tertiary amine) capable of generating radicals upon reaction with an organic peroxide.

The following descriptions are given for illustration of the invention and are not intended to be limiting thereof.

### EXAMPLES

### Examples 1 to 3 and Comparative Examples 1 to 6

Compositions of Examples 1 to 3 and Comparative Examples 1 to 6 were prepared according to prescriptions shown below using diluting solvents shown in Table 1.

| Dental Composition A | |
|---|---|
| 2,2-Bis[methacryloyloxypolyethoxyphenyl]propane (commercial name: D-2.6E, made by Shin-Nakamura Chemical Co., Ltd.) | 6 wt% |
| Neopentyl glycol dimethacrylate | 3 |
| 10-Methacryloyloxydecyl dihydrogen phosphate | 1 |
| Dibenzoyl peroxide | 1 |
| 2,6-Di-tert-butylhydroxytoluene | 0.02 |
| Diluting solvent | 89.0 |

The following tests 1 and 2 were conducted using the above dental composition A to evaluate the stability of dibenzoyl peroxide and the weight reduction caused by evaporation of the diluting agents.

### Test 1

A polypropylene container with a cap (measurements: 20φ x 40 mm, wall thickness: 0.5 mm) was filled with 10 cm³ of Dental Composition A, and the container with the contents was kept at 50°C for 2 days. The amounts of dibenzoyl peroxide before and after the storage were determined by high-performance liquid chromatography to give the residual ratio of dibenzoyl peroxide after storage of 2 days at 50°C. For the determination, an internal standard of 0.1% biphenyl had been added to Dental Composition A. Table 1 shows the calculation results of the residual ratios.

### Test 2

In the same manner as in Test 1, Dental Composition A filled in a plastics container was stored at 50°C for 2 days, and the weight reduction, caused by evaporation of the diluting solvent which permeated through the polypropylene container, of Composition A was measured. The percentages of weight reduction were calculated and shown in Table 1.

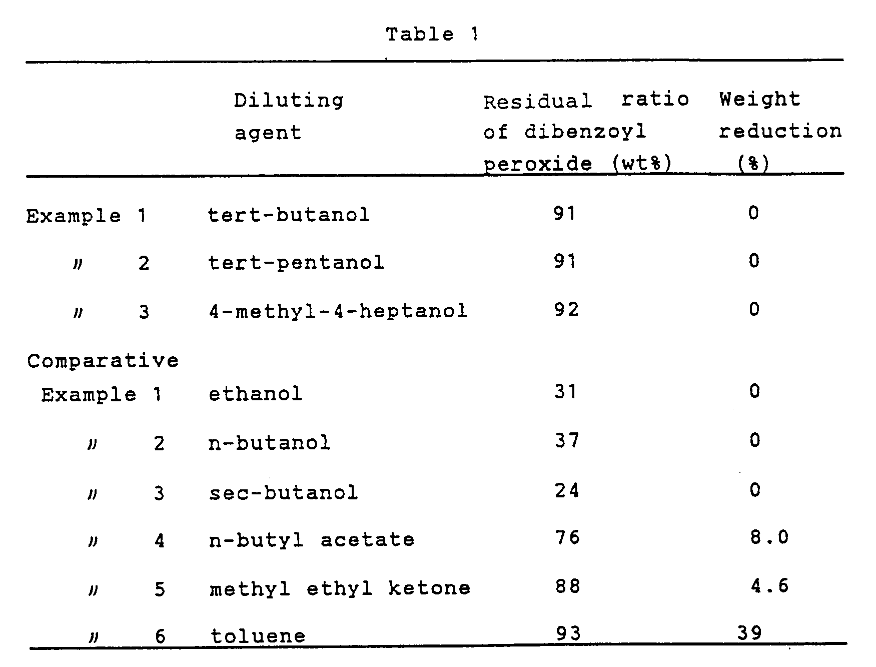

### Comparative Examples 7 to 9

Dental Compositions A with diluting agents n-butyl acetate (Comparative Example 7), methyl ethyl ketone (Comparative Example 8) and toluene (Comparative Example 9) were each filled in a 15-cm³ glass container in a volume of 10 cm³, and the containers were stoppered and kept at 50°C. After one day, all three compositions turned white turbid, indicating evidently that the monomer had polymerized.

## Claims

1. A dental composition comprising a radical-polymerizable monomer and an organic peroxide, which are dissolved in a volatile tertiary alcohol, said composition being packaged in an oxygen-permeable plastics container.

2. The dental composition of Claim 1, wherein said radical-polymerizable monomer is a (meth)acrylate monomer.

3. The dental composition of Claim 1 or Claim 2, wherein said organic peroxide is dibenzoyl peroxide.

4. The dental composition of any preceding claim, wherein said tertiary alcohol is tertiary butanol.

## Patentansprüche

1. Dentalzusammensetzung, enthaltend ein radikalpolymerisierbares Monomer und ein organisches Peroxid, die in einem flüchtigen tertiären Alkohol gelöst sind, wobei die Zusammensetzung in einem sauerstoffdurchlässigen Kunststoffbehälter verpackt ist.

2. Dentalzusammensetzung nach Anspruch 1, wobei es sich bei dem radikalpolymerisierbaren Monomeren um ein (Meth)acrylat-Monomeres handelt.

3. Dentalzusammensetzung nach Anspruch 1 oder 2, wobei es sich bei dem organischen Peroxid um Dibenzoylperoxid handelt.

4. Dentalzusammensetzung nach einem der vorstehenden Ansprüche, wobei es sich beim tertiären Alkohol um tert.-Butanol handelt.

## Revendications

1. Une composition dentaire comprenant un monomère polymérisable par mécanisme radicalaire et un peroxyde organique qui sont dissous dans un alcool tertiaire volatil, ladite composition étant emballée dans un récipient en plastique perméable à l'oxygène.

2. La composition dentaire de la revendication 1, dans laquelle ledit monomère polymérisable par mécanisme radicalaire est un (méth)acrylate monomère.

3. La composition dentaire de la revendication 1 ou de la revendication 2, dans laquelle ledit peroxyde organique est le peroxyde de dibenzoyle.

4. La composition dentaire de l'une quelconque des revendications précédentes, dans laquelle ledit alcool tertiaire est le butanol tertiaire.
